# EUROPEAN PATENT SPECIFICATION

(11) **EP 1 769 731 B1**
(45) Date of publication and mention of the grant of the patent: **11.03.2009**
(21) Application number: 05386023.5
(22) Date of filing: 29.09.2005
(51) Int. Cl.: A61B 1/303, A61B 1/015

(54) **Vaginal speculum arrangement**
Vaginalspekulum
Spéculum vaginal

(43) Date of publication of application: 04.04.2007
(73) Proprietor: Forth-Photonics Ltd, 20-22 Bedford Row WC1R 4JS London (GB)
(72) Inventor: Balas, Konstantinos, 15341 Ag. Paraskevi (GR)
(74) Representative: Spencer, Matthew Peter

(56) References cited:
- WO-A-93/19678
- WO-A-20/05055819
- FR-A- 2 328 440
- US-A- 3 789 829
- US-A- 5 458 595
- US-A1- 2002 197 728
- US-A1- 2003 207 250
- US-A1- 2003 225 313
- US-A1- 2005 090 751

## Description

### SUMMARY OF THE INVENTION

The present invention relates to a vaginal speculum embodying an applicator for the uniform delivery of a standardized dose of a liquid diagnostic marker onto the woman's lower genital tract. The applicator comprises of a marker container and a mechanism for transferring a desirable quantity of its content to an injection probe for dispensing the marker onto the tissue surface. The probe may be a nozzle generating a desirable injection pattern, depending on the location of the tissues to be examined. The cross section of the injection probe is substantially smaller than the rear opening of the speculum, so that the monitoring of the optical effects provoked to the tissue by the marker and the insertion of treatment tools is not obstructed.

The probe may be affixed to an extension rod, which may be mechanically coupled with the speculum blades, in such a way that the longitudinal axis of the probe and consequently the injection direction remains stable, independently from the actual opening angle of the blades, determined by the anatomy of the vaginal wall. Optical, electronic imaging means, illumination means and treatment tools may be mounted onto the extension rod, which rod may be detachably attached to mechanical positioning systems or to imaging devices used in colposcopy.

The disclosed speculum arrangement may be used as a tool for diagnostic and screening examinations and for the treatment of cervical and vaginal neoplasias.

### PRIOR ART

Detection and identification of pathologic alterations of the woman's low genital track (cervix of the uterus, vagina) involves a series of medical procedures including screening tests (pap-test), tissue examination with the aid of a microscope (colposcopy), biopsy sampling and histology. An abnormal pap-test is followed by colposcopy, where the vagina is opened with the aid of a speculum to allow tissue visualization with the aid of a microscope. In colposcopy, a number of diagnostic markers are applied topically, which alter the optical properties of the tissue, depending on the pathology. Particularly, application of 3-5% acetic acid solution provokes a reversible whitening of the abnormal tissue areas. It has been proved that the degree and the duration of the whitening effect correlates well with the neoplasia grade. The provoked contrast enhancement between normal and abnormal areas provide a valuable means for assisting colposcopic diagnosis and for locating abnormal areas for biopsy sampling and treatment. There is a considerable confidence for the diagnostic value of these diagnostic markers, which has been developed during the 70 years usage of these tests in the clinical practice.

The employment of marker-based *in vivo* tests as an alternative to the *in vitro* pap test for screening cervical pathology has increased in recent years. Marker-based *in vivo* tests employ a procedure similar to the colposcopy procedure, but typically are performed without the use of a microscope (colposcope). The vagina is opened with the aid of a speculum, which is followed by the application of acetic acid solution onto tissue surface and naked-eye monitoring of the marker-induced alterations in the colour of the examined tissue. This technique is known as speculoscopy. In contrast to the pap-test, speculoscopy offers diagnostic results immediately, which enable the biopsy sampling and/or the treatment of the lesion even during the same consultation.

One main drawback of both colposcopy and speculoscopy arises from the fact that the quantity of applied marker is not standardized, while the marker administration means and procedures do not ensure its uniform application over the entire area of the examined tissue. In addition, the injection means employed obstruct the rear opening of the speculum, not allowing the monitoring of the effects provoked by the marker during its application and due to this fact critical diagnostic information is missed. Typically, an uncontrolled volume of the marker is applied either by washing the tissue with the aid of a cotton brush, moistened with acetic acid solution, or with the aid of a general purpose, hand held atomizer, which delivers a random quantity of the marker remotely. In some cases more than one injection are performed in a repetitive manner during the evolution of the acetowhitening phenomenon in order to achieve better contrast.

Clinical research, conducted by the inventors of the present invention, has shown that the monitoring of the effects provoked by the marker, during and after its application, has a great diagnostic value. The same research has also shown that the concentration and the quantity of the marker solution, applied onto the examined tissue are very critical since for a given pathology, different marker doses generate different optical effects, which may cause misdiagnosis. Particularly, for a given tissue pathology, an insufficient marker dose may cause in cancerous lesions an acetowhitening pattern similar to the one provoked by an optimum marker quantity in inflammations and in low grade neoplasias. Similarly, a high marker dose can cause an acetowhitening pattern in inflammations and low grade precancerous lesions typically found in cancerous lesions. Consequently, the lack of an arrangement enabling the standardization of the marker quantity applied onto the tissue surface may result in false positive and/or false negative results, thus, diminishing the diagnostic performance of these tests in terms of both sensitivity and specificity.

A number of prior art documents disclose various speculum arrangements with imaging and illuminations means integrated with a speculum, but they are characterized by the lack of injection means for applying uniformly a standardized quantity of a diagnostic marker, while simultaneously allowing for the inspection of the optical effects produced by the latter.

Such prior art documents include GB214913 and GB191027965. These documents disclose a vaginal speculum with incorporated fluid injection means. The purpose of fluid injection means, as described in these documents, is for washing the woman's low genital tract and it does not offer any standardization of the injected liquid. It is worth noticing that in these prior art documents, washing does not employ a diagnostic marker and therefore it is not intended to assist diagnosis and screening. More importantly, it does not allow for the visualization of the area of interest, since the whole inner space of the speculum is occupied by the fluid injection means and no free space is available allowing observation and insertion of treatment tools.

Other prior art documents disclose vaginal specula with integrated illumination means for illuminating the vagina. Such specula are disclosed, e.g., in documents GB1408382, US3762400, US3851642. These vaginal specula are intended for the medical examination of the vagina, but they are not accompanied with integrated fluid injection means, necessary for a diagnostic medical examination of the vagina wherein the uniform application of a standard volume of a diagnostic marker is necessary.

Other prior art described in documents WO9007299, WO9728753, US4210133 and US4046140, discloses vaginal specula with an integrated microscope or camera for observing and/or for capturing images of the cervical tissue. In the described implementations the microscopes or cameras are located within the blades not allowing the insertion of tools for biopsy sampling and treatment simultaneously with the inspection with the aid of a microscope or camera. In addition, the instruments disclosed in the foregoing documents do not allow the injection of diagnostic markers. Finally, the prior art document US20040122327 discloses an uteroscope arrangement including a panoramic lens for viewing the entire uterine cavity in one image that is mounted on an elongated shaft for insertion into the patient's uterus. One or more transparent inflatable balloons are mounted on the elongated shaft surrounding the optical imaging system. An instrument channel is provided in the shaft of the uteroscope for insertion of instruments, such as a suction tube, external to or in between the transparent inflatable balloons.

US 2005/090751 discloses a speculum having an injection means for controlled spraying of tissue.

### OBJECTS OF THE INVENTION

The object of the present invention is to provide a speculum arrangement as defined by claim 1, integrating means for dispensing uniformly a standardised marker volume, while simultaneously allowing for the visualization and monitoring of the provoked optical effects, for diagnostic and screening purposes and the insertion of treatment tools into the vaginal canal, for biopsy sampling and treatment.

It is another object of the present invention to provide a speculum arrangement further integrating optical, electronic imaging, and illumination means with a speculum arrangement embodying a diagnostic marker injection mechanism, while simultaneously allowing for the insertion of treatment tools into the vaginal canal.

It is still another object of the invention to provide a speculum arrangement with an extension shaft, which may be connected with a mechanical support and positioning means, including the ones employed in imaging devices used in colposcopy, for the support and stabilization of the speculum and hands free operation.

### BRIEF DESCRIPTION OF THE DRAWINGS

Figure 1 illustrates a vaginal speculum arrangement composed by blades and handles, an extension rod mechanically coupled with the blades, and injection probe affixed onto the rod, a liquid marker container, and hydraulic means to enable injection of the marker.
Figure 2 illustrates a vaginal speculum arrangement composed of blades and handles, an extension rod mechanically coupled with the blades, and a mechanical support attached to a platform such as the ground or to the examination bed and connected detachably with the extension shaft. Onto the mechanical support and in the vicinity of its connection point with the extension shaft, the following components are mounted: an injection probe, a light source with a removable polarizer and removable rotating imaging polarizer, optical filter means and image magnifying optics.
Figure 3 illustrates a rear-view of a speculum in accordance with the teachings of the present invention.
Figure 4 illustrates a needle nozzle with a needle having an outside diameter sized to maximize the field-of-view through the rear aperture of a speculum in accordance with the teachings of the present invention. A coupling mechanism is used for connection of the needle nozzle with the tube providing a channel through which the marker flows.
Figure 5 illustrates an apparatus for securing a speculum shaft onto an optical imaging system, or onto a base member.

### DETAILED DESCRIPTION OF THE INVENTION

A Cusco-type speculum is illustrated in the figures for illustrative purposes and those skilled in the art will appreciate the present invention is not limited to such a speculum, but rather is applicable to any kind of speculum having a mechanical arrangement suitable for opening the vagina to enable the visualization of the tissues composing a woman's lower genital track.

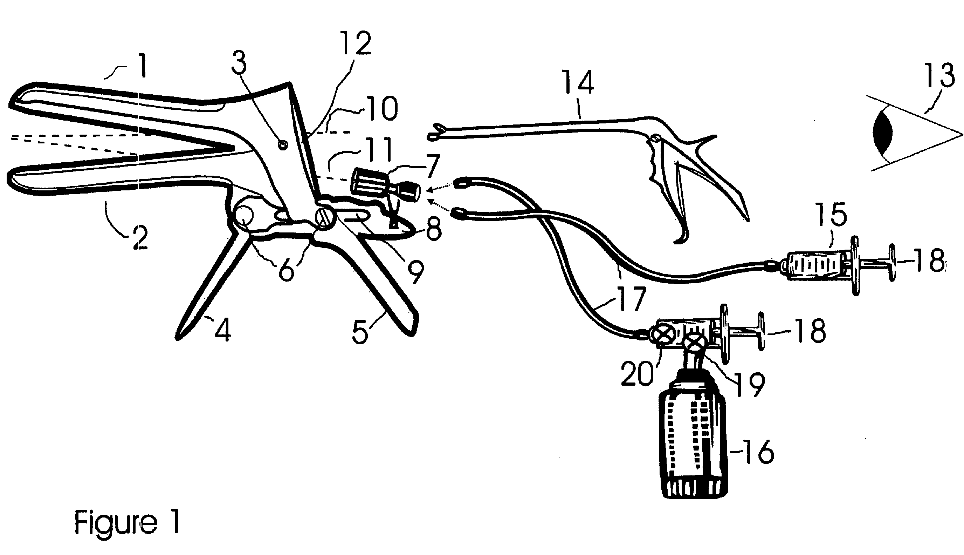

Figure 1 depicts a Cusco-type speculum having two blades (1, 2) connected to each other with the aid of a pivoting joint (3), located at the rear part of the blades. Each blade is jointed with the corresponding handle (4, 5) with the aid of a pin (6). The separation distance between the handles (4, 5) becomes maximum when the front parts of the blades (1, 2) are in contact. When the speculum is inserted into the vagina, the blades are in or near contact to each other, for the patient's comfort. After insertion, the handles (4, 5) are approached to each other, separating the blades (1, 2) and opening the vagina. The blade separation is mechanically locked at a desirable position, determined by the anatomy of the tissue. Then the examination follows involving the application of one or more diagnostic markers and the monitoring of the marker-induced alterations in the properties, e.g., the colour, of the tissue. As it has been stated above, the uniform application of a standardized quantity of the diagnostic marker, while simultaneously allowing for the tissue inspection is critical for examination and diagnostic evaluation.

Uniform and simultaneous application of the marker over the entire area of the examined tissues can be achieved with the aid of a liquid injection mechanism, capable of dispensing the marker from a distance. In the case that the cervix of the uterus is examined, and because of its almost circular shape, a preferable injection pattern is conical with a maximum diameter equal with the diameter of the cervix, which is approximately 2.5-3 cm (1 inch).

An injection probe (7) is preferably mounted properly onto a fixed position, so that its injection direction is not affected by the opening angle of the speculum blades (1, 2), which may vary due to the anatomy of the vagina. Such a fixed position cannot be achieved by affixing the injection probe on any of the blades, since by changing their angle the injection direction will change accordingly. Consequently, depending on the blade angle different parts of the tissue will be exposed to a different volume of the marker fluid.

In the case of a Cusco-type speculum the blades open symmetrically around the speculum's pivoting joint (3), thus composing an eligible mount to affix the probe (7) (not according to the invention). The solution, according to the invention, is to affix the injection probe onto an extension shaft (8), which is mechanically coupled with the pins (6) connecting the handles with the blades. The front part of the shaft is jointed with the blade-handle joint of the first blade (4), while the pin of the blade-handle joint of the second blade (5) can slide within a groove (9), formed along the longitudinal axis of the extension shaft (8). This arrangement ensures that the relative position of the longitudinal axis of the probe (10) with the longitudinal symmetry axis of the blade system (11) remains the same for all possible blade angles. Therefore, by properly mounting the injection probe (7) onto the extension shaft (8), its longitudinal axis intersects the central area of the examined tissue in all possible relative positions of the blades (1, 2), thus ensuring uniform application of the marker in various anatomic conditions.

In one embodiment of the present invention, the injection probe (7) is a nozzle remotely delivering a mist of liquid marker droplets of a desirable size onto the surface of the tissue. The cross section of the injection probe (7) is substantially smaller that the rear opening of the blade system (12) and preferably it has a needle nozzle-like shape for the purpose of not obscuring the visualization (13) of the tissue before, during and after injection and for allowing for the insertion of treatment tools (14). The liquid marker is transmitted to the injection probe (7) from a marker container (15, 16) either by permanently or detachably connecting these parts to each other, or through a tube (17) connecting these parts either permanently or detachably. The injection of the fluid is achieved with the aid of hydraulic pressure manually or otherwise applied.

In one embodiment of the present invention, the container and the hydraulic means comprise a syringe with a container (15) and a piston (18). In another embodiment of the present invention, the container is bottle (16) and the hydraulic means is a tube with two one-way valves (19) and a piston (18). When the piston (18) is pulled out, the liquid fills-up the tube enclosing the piston with a desirable quantity of marker liquid and the valve of the bottle (19) closes. By pushing the piston in, the tube valve (20) opens, the bottle valve (19) closes and the liquid is injected from the injection probe (7). In one embodiment of the invention, more than one marker staining different features of diagnostic performance is performed with the arrangement described above either simultaneously or in time sequence.

Clinical investigations conducted by the inventors of the present invention have shown that the optimum quantity of the marker is a volume of between about 2.5ml and 3.5 ml. This volume ensures a sufficient and uniform washing of the entire surface of the cervix to produce the diagnostic optical effect. At the same time, this volume is desirable, since it eliminates unwanted accumulation of marker in excess between the lower blade (20) and the lower part of the examined tissue, which may obscure the visualization of the tissue.

The vaginal speculum arrangement of the present invention, as illustrated in Figure 1, may be manufactured either in part or in whole either from metallic or from synthetic (plastic, Plexiglas) material. The speculum arrangement of the present invention either in part or in whole, may be either re-usable or disposable. In one embodiment, the speculum arrangement comprising the blade and handle system, the extension shaft onto which the nozzle is affixed, the nozzle mechanically coupled with the syringe pre-filled with the marker, is disposable.

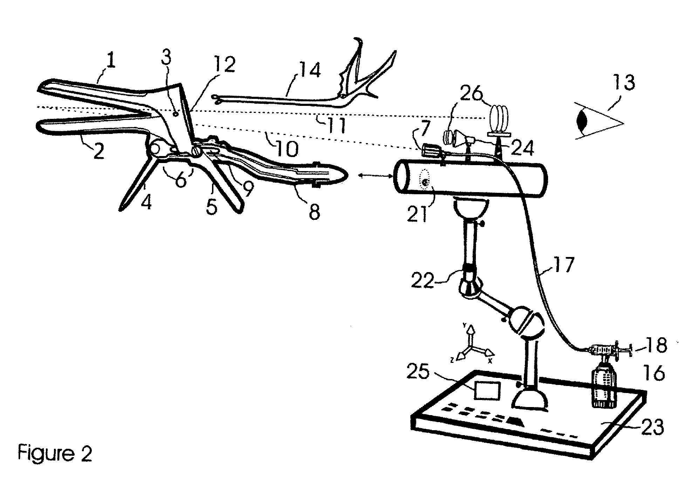

Figure 2 depicts another embodiment of the vaginal speculum arrangement. The length of the extension shaft (8) is determined by the working distance of the optical imaging apparatus employed for the examination the lower part of woman's genital system, such as cameras, colposcopes etc. and combinations thereof. The extension shaft is detachably connected with these imaging apparatuses, with the aid of a locking mechanism (21). The locking mechanism (21) is affixed onto the imaging apparatus and at a proper location so that when the locking mechanism is coupled with the extension shaft, the longitudinal symmetry axis of the blade system (11) coincides substantially with the bisector of the viewing angle (13). In another embodiment of this invention, suitable for speculoscopy use, the locking mechanism (21) is mounted on a mechanical support, which in turn is either affixed onto the examination bed or includes a base (23) placed on the ground. The mechanical support may be an articulating arm (22) to facilitate manipulations for the connection of the speculum shaft (8) with the locking mechanism (21).

Onto the mechanical support (22) and in the vicinity of its connection point with the extension shaft, the following components may be mounted: an injection probe (7), connected with a marker container (16, 15) either directly or though a tube (17) and hydraulic means for enabling injection, all having the specifications described above with respect to Figure 1, a light source (24) with a power supply (25) and at least one of the following optical elements (26) interposed in the illumination and imaging ray paths: magnifying and focusing optics, filters and polarizers. The optical elements (26) may be mounted in a removable manner from the path of the rays, by titling them left or right. The polarizers may be affixed on a mount allowing the rotation of their polarization axes.

The cross section of the light source and illumination optics (24, 26) is substantially smaller than the rear optical aperture of the blade system (12) for the purpose of not obscuring the visualization of the tissue.

The light source (24) may be a halogen lamp and/or a LED lamp or other suitable light source. When the polarization axis of the imaging polarizer becomes, after rotation, vertical with the polarization axis of the light source, then the surface reflection (glare) is eliminated, resulting in a substantial improvement of the perceived contrast. This facilitates the detection and monitoring of features of diagnostic importance. The perceived contrast is further enhanced with the aid of an optical filter and image magnifying means (26).

Once the extension shaft (8) is connected with the mechanical support, the longitudinal axis of the injection probe (12) may have a fixed relative position with the longitudinal axis (11) of the blade system, ensuring that the former intersects the central area of the tissue and the uniform application of the marker onto the entire area of the examined tissue.

The vaginal speculum arrangement of the current invention, illustrated in figure 2, may be manufactured either in part or in total either from metallic or from synthetic (plastic, Plexiglas) material. The speculum arrangement of the current invention may be in part either re-usable or disposable. In one preferable embodiment of the vaginal arrangement as depicted in Figure 2, the blade-handle system with the extension shaft is disposable and the mechanical mount with the components (in part or in whole) mounted on it, is re-usable.

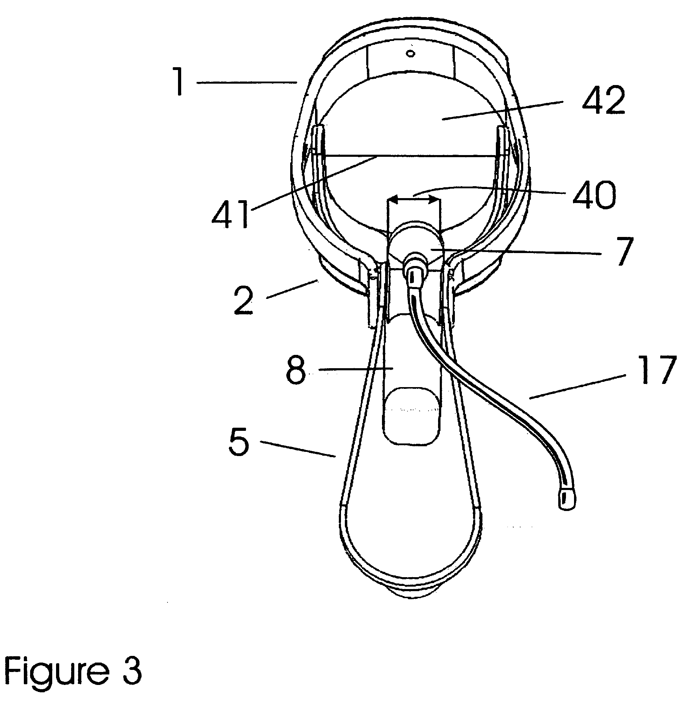

Figure 3 illustrates a rear-view of the joined speculum blades (1, 2), the extension shaft (8) and the nozzle (7). The dimensions of the cross section (40) of the nozzle is substantially smaller than the dimensions of the cross section (41) of a rear aperture (42) of the blade system, thus allowing for the visualization of the examined area before, during, and after the injection of the marker.

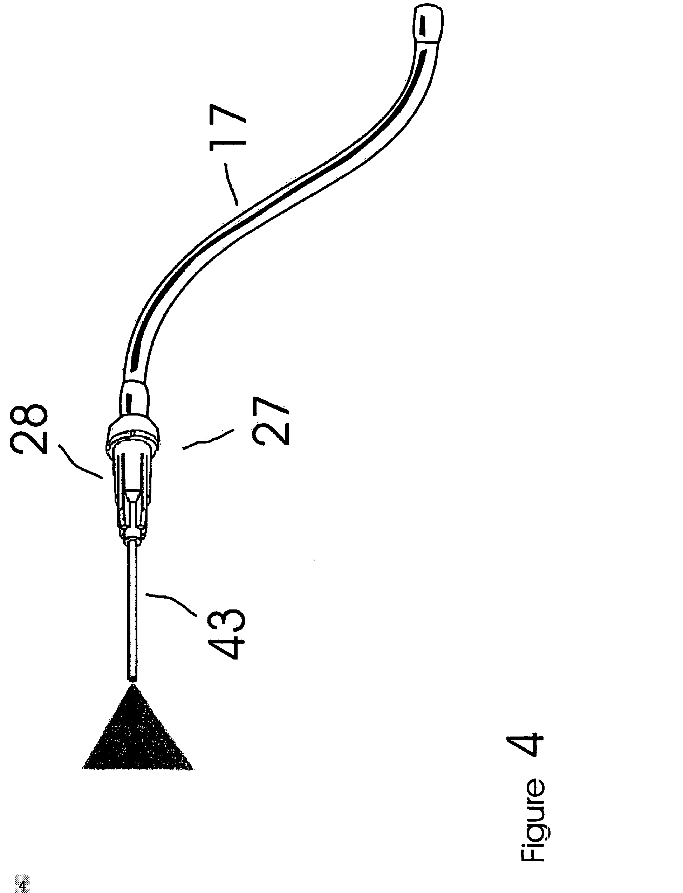

Figure 4 illustrates a needle nozzle (27), with the needle (43) having an outside diameter sized to maximize the field-of-view through the rear aperture (42) of the blade system. A coupling mechanism (28) is used for the connection of the needle nozzle (27) with the tube (17) providing a channel for the marker from a container holding to marker to an input orifice of the coupling means (28).

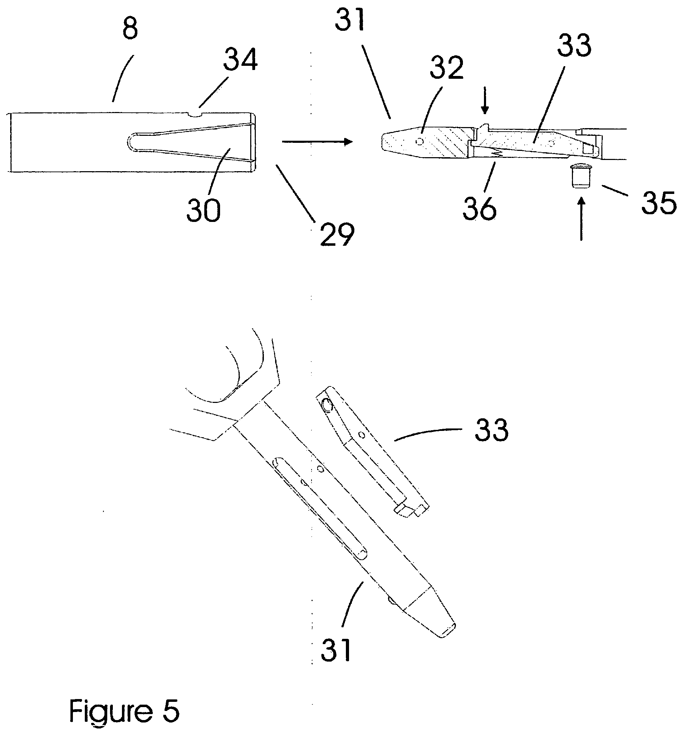

Figure 5 illustrates one embodiment of the shaft (8) in more detail. Those skilled in the art will appreciate the shaft (8) is not limited to the embodiment illustrated and other shaft configurations are possible. The shaft (8) illustrated in Figure 5 is well suited for use in securing the speculum shaft onto an optical imaging system (26), onto a base member (23), or both. In the illustrated embodiment, the distal end (29) of a speculum shaft (8) includes a conically tapered slot (30) in a bottom side. The conically tapered slot (30) acts as a guide for the proper alignment of the speculum with respect to the external optical system (26). A securing mechanism engages with the distal end (29) of the shaft (8) with an extension pin (31) that has a dowel pin (32) having a longitudinal axis perpendicular to the longitudinal axis of the extension pin (31). The position of the dowel pin (32) determines the displacement of the speculum from the external optical system. The distal end (29) of the shaft (8) engages with the extension pin (31) using a spring-loaded, cam action wedge (33). The distal end (29) of the shaft (8) also includes a receptacle slot (34) to mate with the cam action wedge (32).

In operation, the shaft (8) is moved towards the optical system to urge the dowel pin (32) into contact with the conically tapered groove in the shaft (8) until the cam action wedge (33) mates with the receptacle slot (34) in the shaft (8).

The shaft (8) is unlocked from the dowel pin (32) by pressing on a release button (35) which has the effect of engaging with the cam action wedge (32). In this state, the receptacle slot (34) is devoid of a locking member and the shaft (8) can be removed. The cam action wedge (32) and the release button (35) are returned to their normal states due to the action of the spring (36) housed in the engagement pin (31).

## Claims

1. A vaginal speculum arrangement (12) comprising,
a mechanical support having a shaft (8) with a first shaft end and
a second shaft end detachably coupled to an injection mechanism for dispensing a diagnostic marker onto the surface of the examined tissue having an injection probe (7) having a longitudinal axis (11), a marker container (15, 16) and a means for enabling injection of the marker (18) wherein the dimensions of a cross section of the injection probe (7) are substantially smaller than the dimensions of a cross section of a rear aperture of the vaginal speculum arrangement (12),
**characterised in that** the vaginal speculum arrangement comprises a blade system for opening the vagina having a first blade (1) and a second blade (2) connected by a pivoting joint (3) and being positionable relative to each other in a plurality of angles and a longitudinal axis between a distal portion and a proximate portion of each of the first and second blades
wherein the first shaft end is mechanically coupled with the blade system and further **characterised in that** said first shaft end of said shaft is jointed with a blade-handle (5) joint of the first blade (1), and a pin (6) of the blade-handle (4) joint of the second blade (2) moves within a groove (9), formed along a longitudinal axis of said extension shaft (8) to ensure that the relative position of the longitudinal axis of the injection probe (11) and the longitudinal symmetry axis of the blade system remain substantially fixed for each of the plurality of angles between the first (1) and second (2) blades, and
wherein the injection probe (7) allows for a substantially homogenous application of the marker on a desired area of the examined vaginal or cervical tissue, irrespective of an opening angle of the first (1) and second (2) blades and allows for observation of the desired area through the rear aperture of the blade system, before, during, and after the injection of the marker.

2. The vaginal speculum arrangement according to claim 1, further **characterised in that** the second shaft end is detachably coupled to an imaging apparatus.

3. The vaginal speculum arrangement according to claim 1, further **characterised in that** the second shaft end is detachably coupled to a base member.

4. A vaginal speculum arrangement according to claim 1 further **characterized in that** said injection probe mounts to a portion of said mechanical support's shaft.

5. The vaginal speculum arrangement of claim 3 further **characterised in that** said base member includes an articulated arm (22) with a first end portion affixed to a base (23) and a second end portion affixed to a locking mechanism (21) of the shaft.

6. The vaginal speculum arrangement of claim 5, further **characterised in that** said locking mechanism (21) includes one of a mechanical locking mechanism, a magnetic locking mechanism, or an electromagnetic locking mechanism.

7. The vaginal speculum arrangement of claim 5 or 6 further **characterised in that** said injection probe (7) is affixed onto said base member in a vicinity of said locking mechanism.

8. The vaginal speculum arrangement of claim 1 further **characterised in that** said injection probe comprises a nozzle.

9. The vaginal speculum arrangement of claim 8 further **characterised in that** said nozzle comprises a needle nozzle (27).

10. The vaginal speculum arrangement of claim 1 further **characterised in that** the injection mechanism further comprises a hydraulic pump means for pumping a predetermined volume of a marker into and through said injection probe.

11. The vaginal speculum arrangement of claim 10, **characterised in that** said predetermined volume of the marker ranges between 2.5ml and 3.5ml.

12. The vaginal speculum arrangement of claim 10, further **characterised in that** said marker is between 3% and 5% acetic acid solution.

13. The vaginal speculum arrangement of claim 1 further **characterised by** further comprising a light source (24).

14. The vaginal speculum arrangement of claim 5 further **characterised by** further comprising a light source (24) affixed to the base member in a vicinity of said shaft's locking mechanism (21).

15. The vaginal speculum arrangement of claim 1, further **characterised by** further comprising an optical element (26).

16. The vaginal speculum arrangement of claim 15, further **characterised in that** the optical element (26) comprises one of a magnifying optical element, a focusable optical element, an optical filter or a polarizer.

17. The vaginal speculum arrangement of any of claims 1 to 16 further **characterised in that** said blade system, said shaft (8) and said injection probe (7) are formed from a metallic material.

18. The vaginal speculum arrangement of claim 17 further **characterised in that** said blade system, said shaft (8) and said injection probe (7) are re-usable.

19. The vaginal speculum arrangement of any of claims 1 to 16, further **characterised in that** said blade system, said shaft (8) and said injection probe (7) each have a portion formed from a polymeric compound.

20. The vaginal speculum arrangement of claim 19, further **characterised in that** said blade system, said shaft (8) and said injection probe (7) are disposable.

## Patentansprüche

1. Vaginoskop-Anordnung (12), umfassend
einen mechanischen Träger mit einem Schaft (8) mit einem ersten Schaftende und
einem zweiten Schaftende, das abnehmbar an eine Injektionseinrichtung zur Abgabe eines diagnostischen Markers auf die Oberfläche des untersuchten Gewebes gekoppelt ist, wobei die Injektionseinrichtung eine Injektionsprobe (7) mit einer Längsachse (11), einen Marker-Behälter (15, 16) und ein Mittel zum Ausführen der Injektion des Markers (18) aufweist, wobei die Abmessung eines Querschnitts der Injektionsprobe (7) im wesentlichen kleiner als die Abmessung eines Querschnitts der hinteren Öffnung der Vaginoskop-Anordnung (12) ist,
**dadurch gekennzeichnet, dass** die Vaginoskop-Anordnung ein Flügelsystem zum Öffnen der Vagina umfasst, das einen ersten Flügel (1) und einen zweiten Flügel (2) aufweist, welche über ein drehbares Gelenk (3) verbunden sind und im Verhältnis zueinander in einer Vielzahl von Winkeln positionierbar sind, sowie eine Längsachse zwischen einem entfernt gelegenen Abschnitt und einem nah gelegenen Abschnitt von jedem des ersten und zweiten Flügels umfasst,
wobei das erste Schaftende mechanisch an das Flügelsystem gekoppelt ist, und ferner **dadurch gekennzeichnet ist, dass** das erste Schaftende des Schafts mit einem Flügelgriff (5)-Gelenk des ersten Flügels (1) verbunden ist, und sich ein Stift (6) des Flügelgriff-Gelenks (4) des zweiten Flügels (2) innerhalb einer Rille (9) bewegt, die entlang einer Längsachse des Schiebeschafts (8) gebildet ist, um zu gewährleisten, dass die relative Position der Längsachse der Injektionsprobe (11) und der Längssymmetrieachse des Flügelsystems im Wesentlichen für jeden der Vielzahl von Winkeln zwischen dem ersten (1) und zweiten (2) Flügel nicht fixiert bleibt, und
wobei die Injektionsprobe (7) ein im wesentlichen gleichmäßiges Auftragen des Markers auf eine gewünschte Fläche des untersuchten Vaginal- oder Cervix-Gewebes ermöglicht, unabhängig von einem Öffnungswinkel des ersten (1) und zweiten (2) Flügels, und die Beobachtung der gewünschten Fläche über die Hinteröffnung des Flügelsystems vor, während und nach der Injektion des Markers ermöglicht.

2. Vaginoskop-Anordnung nach Anspruch 1, ferner **dadurch gekennzeichnet, dass** das zweite Schaftende abnehmbar an einen bildgebenden Apparat gekoppelt ist.

3. Vaginoskop-Anordnung nach Anspruch 1, ferner **dadurch gekennzeichnet, dass** das zweite Schaftende abnehmbar an ein Basiselement gekoppelt ist.

4. Vaginoskop-Anordnung nach Anspruch 1, ferner **dadurch gekennzeichnet, dass** die Injektionsprobe an einen Abschnitt des Schaftes des mechanischen Trägers angebracht ist.

5. Vaginoskop-Anordnung nach Anspruch 3, ferner **dadurch gekennzeichnet, dass** das Basiselement einen Gelenkarm (22) mit einem ersten Endabschnitt, der an einer Basis (23) angebracht ist, und einen zweiten Endabschnitt, der an einem Verriegelungsmechanismus (21) des Schafts angebracht ist, umfasst.

6. Vaginoskop-Anordnung nach Anspruch 5, ferner **dadurch gekennzeichnet, dass** der Verriegelungsmechanismus (21) einen Verriegelungsmechanismus aus einem mechanischen Verriegelungsmechanismus, einem magnetischen Verriegelungsmechanismus oder einem elektromagnetischen Verriegelungsmechanismus umfasst.

7. Vaginoskop-Anordnung nach Anspruch 5 oder 6, ferner **dadurch gekennzeichnet, dass** die Injektionsprobe (7) auf dem Basiselement in der Nähe des Verriegelungsmechanismus befestigt ist.

8. Vaginoskop-Anordnung nach Anspruch 1, ferner **dadurch gekennzeichnet, dass** die Injektionsprobe eine Düse umfasst.

9. Vaginoskop-Anordnung nach Anspruch 8, ferner **dadurch gekennzeichnet, dass** die Düse eine Düsennadel (27) umfasst.

10. Vaginoskop-Anordnung nach Anspruch 1, ferner **dadurch gekennzeichnet, dass** die Injektionseinrichtung ferner Hydraulikpumpen-Mittel zum Pumpen eines vorher festgelegten Volumens eines Markers in und durch die Injektionsprobe umfasst.

11. Vaginoskop-Anordnung nach Anspruch 10, ferner **dadurch gekennzeichnet, dass** das vorher festgelegte Volumen des Markers zwischen 2,5 ml und 3,5 ml liegt.

12. Vaginoskop-Anordnung nach Anspruch 10, ferner **dadurch gekennzeichnet, dass** der Marker eine Essigsäure-Lösung darstellt, die zwischen 3 % und 5 % liegt.

13. Vaginoskop-Anordnung nach Anspruch 1, ferner **dadurch gekennzeichnet, dass** sie weiterhin eine Lichtquelle (24) umfasst.

14. Vaginoskop-Anordnung nach Anspruch 5, ferner **dadurch gekennzeichnet, dass** sie weiterhin eine Lichtquelle (24) umfasst, die an dem Basiselement in der Nähe des Verriegelungsmechanismus (21) des Schafts angebracht ist.

15. Vaginoskop-Anordnung nach Anspruch 1, ferner **dadurch gekennzeichnet, dass** sie weiterhin ein optisches Element (26) umfasst.

16. Vaginoskop-Anordnung nach Anspruch 15, ferner **dadurch gekennzeichnet, dass** das optische Element (26) ein Element von einem vergrößernden optischen Element, einem fokussierbaren optischen Element, einem optischen Filter oder einem Polarisator ist.

17. Vaginoskop-Anordnung nach einem der Ansprüche 1 bis 16, ferner **dadurch gekennzeichnet, dass** das Flügelsystem, der Schaft (8) und die Injektionsprobe (7) aus metallischem Material gebildet sind.

18. Vaginoskop-Anordnung nach Anspruch 17, ferner **dadurch gekennzeichnet, dass** das Flügelsystem, der Schaft (8) und die Injektionsprobe (7) wiederverwendbar sind.

19. Vaginoskop-Anordnung nach einem der Ansprüche 1 bis 16, ferner **dadurch gekennzeichnet, dass** das Flügelsystem, der Schaft (8) und die Injektionsprobe (7) jeweils einen Abschnitt aufweisen, der aus einer Polymerverbindung gebildet ist.

20. Vaginoskop-Anordnung nach Anspruch 19, ferner **dadurch gekennzeichnet, dass** das Flügelsystem, der Schaft (8) und die Injektionsprobe (7) wegwerfbar sind.

## Revendications

1. Agencement de speculum vaginal (12) comprenant:
un support mécanique ayant un arbre (8) avec une première extrémité d'arbre et
une seconde extrémité d'arbre couplée amoviblement à un mécanisme d'injection pour distribuer un marqueur diagnostic sur la surface du tissu examiné ayant une sonde d'injection (7) ayant un axe longitudinal (11), un contenant de marqueur (15, 16) et un moyen pour permettre l'injection du marqueur (18), où les dimensions d'une section transversale de la sonde d'injection (7) sont sensiblement plus petites que les dimensions d'une section transversale d'une ouverture arrière de l'agencement de speculum vaginal (12),
**caractérisé en ce que** l'agencement de speculum vaginal comprend un système à lames pour ouvrir le vagin ayant une première lame (1) et une seconde lame (2) reliées par un joint pivotant (3) et étant positionnables l'une relativement à l'autre selon plusieurs angles et un axe longitudinal entre une portion distale et une portion proximale de chacune des première et seconde lames,
où la première extrémité d'arbre est couplée mécaniquement au système à lames, et **caractérisé en outre en ce que** la première extrémité d'arbre dudit arbre est reliée à une poignée à lames (5) liée à la première lame (1), et un axe (6) de la poignée à lames (4) relié à la seconde lame (2) se déplace dans une rainure (9), ménagée le long d'un axe longitudinal dudit arbre d'extension (8) pour assurer que la position relative de l'axe longitudinal de la sonde d'injection (11) et l'axe de symétrie longitudinal du système à lames reste sensiblement fixe pour chacun d'une pluralité d'angles entre les première (1) et seconde (2) lames, et
où la sonde d'injection (7) permet une application sensiblement homogène du marqueur sur une zone souhaitée du tissu vaginal ou cervical examiné, quelque soit l'angle de l'ouverture des première (1) et seconde (2) lames et permet l'observation de la zone souhaitée à travers l'ouverture arrière du système à lames, avant, pendant et après l'injection du marqueur.

2. Agencement de speculum vaginal selon la revendication 1, **caractérisé en outre en ce que** la seconde extrémité d'arbre est couplée amoviblement à un appareil d'imagerie.

3. Agencement de speculum vaginal selon la revendication 1, **caractérisé en outre en ce que** la seconde extrémité d'arbre est couplée amoviblement à un élément de base.

4. Agencement de speculum vaginal selon la revendication 1, **caractérisé en outre en ce que** ladite sonde d'injection est montée sur une portion dudit arbre du support mécanique.

5. Appareil de speculum vaginal selon la revendication 3, **caractérisé en outre en ce que** ledit élément de base comprend un bras articulé (22) avec une première portion d'extrémité fixée à une base (23) et une seconde portion d'extrémité fixée à un mécanisme de verrouillage (21) de l'arbre.

6. Agencement de speculum vaginal selon la revendication 5, **caractérisé en outre en ce que** ledit mécanisme de verrouillage (21) comprend un parmi un mécanisme de verrouillage mécanique, un mécanisme de verrouillage magnétique ou un mécanisme de verrouillage électromagnétique.

7. Agencement de speculum vaginal selon la revendication 5 ou 6, **caractérisé en outre en ce que** ladite sonde d'injection (7) est fixée sur ledit élément de base au voisinage dudit mécanisme de verrouillage.

8. Agencement de speculum vaginal selon la revendication 1, **caractérisé en outre en ce que** ladite sonde d'injection comprend une buse.

9. Agencement de speculum vaginal selon la revendication 8, **caractérisé en outre en ce que** ladite buse comprend une buse d'aiguille (27).

10. Agencement de speculum vaginal selon la revendication 1, **caractérisé en outre en ce que** le mécanisme d'injection comprend en outre un moyen de pompe hydraulique pour pomper un volume prédéterminé d'un marqueur dans et à travers ladite sonde d'injection.

11. Agencement de speculum vaginal selon la revendication 10, **caractérisé en ce que** ledit volume prédéterminé du marqueur s'étend entre 2,5 ml et 3,5 ml.

12. Agencement de speculum vaginal selon la revendication 10, **caractérisé en outre en ce que** ledit marqueur est une solution d'acide acétique entre 3% et 5%.

13. Agencement de speculum vaginal selon la revendication 1, **caractérisé en outre en ce qu'**il comprend en outre une source de lumière (24).

14. Agencement de speculum vaginal selon la revendication 5, **caractérisé en** outre en comprenant en outre une source de lumière (24) fixée à l'élément de base au voisinage dudit mécanisme de verrouillage (21) de l'arbre.

15. Agencement de speculum vaginal selon la revendication 1, **caractérisé en outre en ce qu'**il comprend un élément optique (26).

16. Agencement de speculum vaginal selon la revendication 15, **caractérisé en outre en ce que** l'élément optique (26) comprend un parmi un élément optique d'agrandissement, un élément optique focalisable, un filtre optique ou un polariseur.

17. Agencement de speculum vaginal selon l'une quelconque des revendications 1 à 16, **caractérisé en outre en ce que** ledit système à lames, ledit arbre (8) et ladite sonde d'injection (7) sont réalisés en un matériau métallique.

18. Agencement de speculum vaginal selon la revendication 17, **caractérisé en outre en ce que** ledit système à lames, ledit arbre (8) et ladite sonde d'injection (7) sont réutilisables.

19. Agencement de speculum vaginal selon l'une quelconque des revendications 1 à 16, **caractérisé en outre en ce que** ledit système à lames, ledit arbre (8) et ladite sonde d'injection (7) ont chacun une portion formée à partir d'un composé de polymère.

20. Agencement de speculum vaginal selon la revendication 19, **caractérisé en outre en ce que** ledit système à lames, ledit arbre (8) et ladite sonde d'injection (7) peuvent être mis au rebut.
